Europäisches Patentamt

European Patent Office

Office européen des brevets

(19)

(11) EP 0 787 989 A2

(12) EUROPÄISCHE PATENTANMELDUNG

(43) Veröffentlichungstag:
06.08.1997 Patentblatt 1997/32

(51) Int Cl.⁶: G01N 33/96, G01N 33/86

(21) Anmeldenummer: 97890017.3

(22) Anmeldetag: 29.01.1997

(84) Benannte Vertragsstaaten:
AT BE CH DE DK ES FI FR GB IT LI NL SE

(30) Priorität: 30.01.1996 AT 171/96

(71) Anmelder: IMMUNO Aktiengesellschaft
A-1221 Wien (AT)

(72) Erfinder:
• Lang, Hartmut, Dr.
2560 Grillenberg (AT)
• Moritz, Berta, Dr.
1030 Wien (AT)

(74) Vertreter: Alge, Daniel et al
Patentanwälte
Sonn, Pawloy, Weinzinger & Wolfram
Riemergasse 14
1010 Wien (AT)

Bemerkungen:
Der Anmelder hat nachträglich ein Sequenzprotokoll eingereicht und erklärt, dass dieses keine neuen Angaben enthält.

(54) **Verfahren zur Herstellung eines standardisierten Plasmapools für diagnostische Zwecke**

(57) Beschrieben wird ein Verfahren zur Herstellung eines standardisierten Plasmapools für diagnostische Zwecke, wobei ausschließlich Plasmaspenden mit normaler APC-Response als Ausgangsmaterial eingesetzt werden.

SEQUENZPROTOKOLL

(1) ALLGEMEINE ANGABEN:

    (i) ANMELDER:
        (A) NAME: IMMUNO Aktiengesellschaft
        (B) STRASSE: Industriestrasse
        (C) ORT: Vienna
        (D) BUNDESLAND: Vienna
        (E) LAND: Austria
        (F) POSTLEITZAHL: A-1221
        (G) TELEFON: ++43-1-20100-0
        (H) TELEFAX: ++43-1-20100-0

    (ii) BEZEICHNUNG DER ERFINDUNG:
Verfahren zur Herstellung eines standardisierten Plasmapools
fuer diagnostische Zwecke

    (iii) ANZAHL DER SEQUENZEN: 15

    (iv) COMPUTER-LESBARE FASSUNG:
        (A) DATENTRÄGER: Tape
        (B) COMPUTER: IBM PC compatible
        (C) BETRIEBSSYSTEM: PC-DOS/MS-DOS
        (D) SOFTWARE: PatentIn Release #1.0,
                     Version #1.30 (EPA)

(2) ANGABEN ZU SEQ ID NO: 1:

    (i) SEQUENZKENNZEICHEN:
        (A) LÄNGE: 13 Aminosäuren
        (B) ART: Aminosäure
        (C) STRANGFORM: Einzelstrang
        (D) TOPOLOGIE: linear

    (ii) ART DES MOLEKÜLS: Peptid

    (iii) HYPOTHETISCH: NEIN

    (xi) SEQUENZBESCHREIBUNG: SEQ ID NO: 1:

Gly Leu Leu His Asn Tyr Gly Val Tyr Thr Lys Val Ser
1               5                   10

(2) ANGABEN ZU SEQ ID NO: 2:

    (i) SEQUENZKENNZEICHEN:
        (A) LÄNGE: 13 Aminosäuren
        (B) ART: Aminosäure
        (C) STRANGFORM: Einzelstrang
        (D) TOPOLOGIE: linear

    (ii) ART DES MOLEKÜLS: Peptid

(iii) HYPOTHETISCH: NEIN

(xi) SEQUENZBESCHREIBUNG: SEQ ID NO: 2:

Leu Glu Gly Tyr Asp Leu Arg Arg Trp Glu Lys Trp Glu
1               5                   10

(2) ANGABEN ZU SEQ ID NO: 3:

    (i) SEQUENZKENNZEICHEN:
      (A) LÄNGE: 13 Aminosäuren
      (B) ART: Aminosäure
      (C) STRANGFORM: Einzelstrang
      (D) TOPOLOGIE: linear

    (ii) ART DES MOLEKÜLS: Peptid

(iii) HYPOTHETISCH: NEIN

(xi) SEQUENZBESCHREIBUNG: SEQ ID NO: 3:

His Asn Tyr Gly Val Tyr Thr Lys Val Ser Arg Tyr Leu
1               5                   10

(2) ANGABEN ZU SEQ ID NO: 4:

    (i) SEQUENZKENNZEICHEN:
      (A) LÄNGE: 13 Aminosäuren
      (B) ART: Aminosäure
      (C) STRANGFORM: Einzelstrang
      (D) TOPOLOGIE: linear

    (ii) ART DES MOLEKÜLS: Peptid

(iii) HYPOTHETISCH: NEIN

(xi) SEQUENZBESCHREIBUNG: SEQ ID NO: 4:

Phe Gln Asn Val Asp Asp Thr Leu Ala Phe Trp Ser Lys
1               5                   10

(2) ANGABEN ZU SEQ ID NO: 5:

    (i) SEQUENZKENNZEICHEN:
      (A) LÄNGE: 13 Aminosäuren
      (B) ART: Aminosäure
      (C) STRANGFORM: Einzelstrang
      (D) TOPOLOGIE: linear

    (ii) ART DES MOLEKÜLS: Peptid

(iii) HYPOTHETISCH: NEIN

(xi) SEQUENZBESCHREIBUNG: SEQ ID NO: 5:

Gly Gln Glu Thr Leu Val Thr Gly Trp Gly Tyr His Ser
1               5                   10

(2) ANGABEN ZU SEQ ID NO: 6:

    (i) SEQUENZKENNZEICHEN:
        (A) LÄNGE: 14 Aminosäuren
        (B) ART: Aminosäure
        (C) STRANGFORM: Einzelstrang
        (D) TOPOLOGIE: linear

    (ii) ART DES MOLEKÜLS: Peptid

  (iii) HYPOTHETISCH: NEIN

    (xi) SEQUENZBESCHREIBUNG: SEQ ID NO: 6:

Leu Leu Thr Ser Leu Leu Leu Phe Val Ala Thr Trp Gly Ile
1               5                   10

(2) ANGABEN ZU SEQ ID NO: 7:

    (i) SEQUENZKENNZEICHEN:
        (A) LÄNGE: 12 Aminosäuren
        (B) ART: Aminosäure
        (C) STRANGFORM: Einzelstrang
        (D) TOPOLOGIE: linear

    (ii) ART DES MOLEKÜLS: Peptid

  (iii) HYPOTHETISCH: NEIN

    (xi) SEQUENZBESCHREIBUNG: SEQ ID NO: 7:

Tyr Lys Leu Gly Asp Asp Leu Leu Gln Cys His Pro
1               5                   10

(2) ANGABEN ZU SEQ ID NO: 8:

    (i) SEQUENZKENNZEICHEN:
        (A) LÄNGE: 7 Aminosäuren
        (B) ART: Aminosäure
        (C) STRANGFORM: Einzelstrang
        (D) TOPOLOGIE: linear

    (ii) ART DES MOLEKÜLS: Peptid

  (iii) HYPOTHETISCH: NEIN

(xi) SEQUENZBESCHREIBUNG: SEQ ID NO: 8:

His Pro Asn Tyr Ser Lys Ser
1               5


(2) ANGABEN ZU SEQ ID NO: 9:

    (i) SEQUENZKENNZEICHEN:
       (A) LÄNGE: 25 Aminosäuren
       (B) ART: Aminosäure
       (C) STRANGFORM: Einzelstrang
       (D) TOPOLOGIE: linear

    (ii) ART DES MOLEKÜLS: Peptid

   (iii) HYPOTHETISCH: NEIN

    (xi) SEQUENZBESCHREIBUNG: SEQ ID NO: 9:

Leu Asp Ile Lys Glu Val Phe Val His Pro Asn Tyr Ser Lys Ser Thr
1               5                   10              15

Thr Asp Asn Asp Ile Ala Leu Leu His
                20              25


(2) ANGABEN ZU SEQ ID NO: 10:

    (i) SEQUENZKENNZEICHEN:
       (A) LÄNGE: 16 Aminosäuren
       (B) ART: Aminosäure
       (C) STRANGFORM: Einzelstrang
       (D) TOPOLOGIE: linear

    (ii) ART DES MOLEKÜLS: Peptid

   (iii) HYPOTHETISCH: NEIN

    (xi) SEQUENZBESCHREIBUNG: SEQ ID NO: 10:

Thr Leu Val Thr Gly Trp Gly Tyr His Ser Ser Arg Glu Lys Glu Ala
1               5                   10              15


(2) ANGABEN ZU SEQ ID NO: 11:

    (i) SEQUENZKENNZEICHEN:
       (A) LÄNGE: 13 Aminosäuren
       (B) ART: Aminosäure
       (C) STRANGFORM: Einzelstrang
       (D) TOPOLOGIE: linear

    (ii) ART DES MOLEKÜLS: Peptid

   (iii) HYPOTHETISCH: NEIN

(xi) SEQUENZBESCHREIBUNG: SEQ ID NO: 11:

Ile Arg Asp Lys Glu Ala Pro Gln Lys Ser Trp Ala Pro
1               5               10

(2) ANGABEN ZU SEQ ID NO: 12:

    (i) SEQUENZKENNZEICHEN:
        (A) LÄNGE: 14 Aminosäuren
        (B) ART: Aminosäure
        (C) STRANGFORM: Einzelstrang
        (D) TOPOLOGIE: linear

   (ii) ART DES MOLEKÜLS: Peptid

 (iii) HYPOTHETISCH: NEIN

    (xi) SEQUENZBESCHREIBUNG: SEQ ID NO: 12:

Lys Ser Arg Ser Asp Arg Arg Gly Ile Gly Arg Ala Ala Asp
1               5               10

(2) ANGABEN ZU SEQ ID NO: 13:

    (i) SEQUENZKENNZEICHEN:
        (A) LÄNGE: 13 Aminosäuren
        (B) ART: Aminosäure
        (C) STRANGFORM: Einzelstrang
        (D) TOPOLOGIE: linear

   (ii) ART DES MOLEKÜLS: Peptid

 (iii) HYPOTHETISCH: NEIN

    (xi) SEQUENZBESCHREIBUNG: SEQ ID NO: 13:

Met Thr Arg Arg Gly Asp Ser Pro Trp Gln Val Val Leu
1               5               10

(2) ANGABEN ZU SEQ ID NO: 14:

    (i) SEQUENZKENNZEICHEN:
        (A) LÄNGE: 19 Aminosäuren
        (B) ART: Aminosäure
        (C) STRANGFORM: Einzelstrang
        (D) TOPOLOGIE: linear

   (ii) ART DES MOLEKÜLS: Peptid

 (iii) HYPOTHETISCH: NEIN

```
(xi)  SEQUENZBESCHREIBUNG: SEQ ID NO: 14:

Thr Gly Trp Gly Tyr His Ser Ser Arg Glu Lys Glu Ala Lys Arg Asn
1                   5                   10                  15

Arg Thr Phe
```

```
(2)  ANGABEN ZU SEQ ID NO: 15:

     (i)  SEQUENZKENNZEICHEN:
          (A)  LÄNGE: 14 Aminosäuren
          (B)  ART: Aminosäure
          (C)  STRANGFORM: Einzelstrang
          (D)  TOPOLOGIE: linear

     (ii)  ART DES MOLEKÜLS: Peptid

     (iii)  HYPOTHETISCH: NEIN

     (xi)  SEQUENZBESCHREIBUNG: SEQ ID NO: 15:

     Lys Ser Arg Ser Asp Arg Arg Gly Ile Gln Arg Ala Ala Asp
     1               5                   10
```

**Beschreibung**

Die vorliegende Erfindung betrifft ein Verfahren zur Herstellung eines standardisierten Plasmapools für diagnostische Zwecke sowie aus Einzelplasmaspenden hergestellte Qualitätsprodukte, die mittels des erfindungsgemäßen Verfahrens kontrolliert worden sind.

Per Definition enthält ein frisches gepooltes Normalplasma, hergestellt aus Plasmen klinisch gesunder Personen, eine Einheit-Gerinnungsaktivität/ml bzw. 100% der Norm.

Gepooltes Normalplasma, welches beispielsweise aus mehr als 100 Plasmaspenden erhalten wird, garantiert im allgemeinen eine gleichmäßige Verteilung der Plasmafaktoren und Inhibitoren. Die Verteilung der Gerinnungsfaktoren bzw. der Inhibitoren in den entsprechenden Pools folgt im allgemeinen einer Normalverteilung. Die Gerinnungsaktivität solcher Pools erfüllt die Voraussetzung der Basisdefinition für jeden Faktor des Blutgerinnungssystems (siehe z.B. H. Lang, Standardmaterialien in der Hämostaseologie, Lab.med. 12 (1988), p. 293-297). Nur für Globalteste wie die Thromboplastinzeitbestimmung (TPZ) reichen zur Herstellung eines gefrorenen Referenzplasmas mit 100% der Norm 30 Einzelplasmen aus (siehe DIN 58939).

Gerade bei Gerinnungsuntersuchungen stellt die Präzisionskontrolle, also die Erfassung der Ergebnisabweichungen in der Serie von Tag zu Tag, und besonders die Richtigkeit, also das Auffinden des wahren Wertes, einen wichtigen Bestandteil der Laboratoriumsarbeit und auch der medizinischen Routinediagnostik dar. Deshalb ist es von großer Bedeutung, sogenannte Normalplasmen als Standards zur Verfügung zu haben. Zur Präzisionskontrolle der Thromboplastinzeit, der partiellen Thromboplastinzeit, der Thrombinzeit oder anderer Gerinnungstests ist hierzu beispielsweise das *Immunocontrol Plasma Normal*® (IMMUNO AG, Österreich) kommerziell erhältlich. Ein Normalplasma zur Richtigkeitskontrolle der Gerinnungsaktivität von Einzelfaktoren bzw. Inhibitoren wird aus ausgewählten Citratplasmen von mindestens 100 Abnahmen gesunder, österreichischer Spender ohne bakterielle Zusätze hergestellt und ist kommerziell erhältlich als "Reference Plasma 100%" (IMMUNO AG, Österreich). Auch Richtigkeitskontrollen für Globalteste, welche geringfügige, durch den Lyophilisationsschritt abweichende Aktivitäten zeigen können, werden aus Plasmapools von 100 Spenden hergestellt (z.B. "Controll Plasma Normal", IMMUNO AG).

Die Spenden, aus denen die Plasmapools hergestellt werden, können jedoch auch von Personen mit Defekten in bestimmten Gerinnungsfaktoren oder in Inhibitoren der Blutgerinnung stammen. Solche Inhibitor-Defekte betreffen beispielsweise einen erworbenen Mangel an Antithrombin III, Protein C oder Protein S. Bei Auswahl einer gesunden, normalen Spenderpopulation ist die Häufigkeit an Defekten jedoch im allgemeinen so gering, daß es bei Vorliegen größerer Plasmapools zu keinen meßbaren Änderungen in den charakteristischen Parametern kommt.

Gerade durch die neueren Erkenntnisse erlangt der Protein C Pathway eine besondere Bedeutung.

Protein C ist ein Vitamin K-abhängiges Glykoprotein, das in der Leber synthetisiert wird und im Plasma als inaktives Zymogen in einer Konzentration von etwa 4 µg/ml zirkuliert. Dieses Protein wird durch den Thrombin - Thrombomodulin-Komplex an der Oberfläche der Gefäßwand (Endothelium) in eine aktive Serin-Protease (aktiviertes Protein C, APC) umgewandelt. Das Protein C kann aber auch durch nicht physiologische Enzyme, wie beispielsweise Schlangengifte, aktiviert werden.

APC hat fibrinolytische und thrombolytische Eigenschaften, und es besitzt aufgrund seiner Fähigkeit, Faktor Va und Faktor VIIIa proteolytisch abzubauen, auch eine antikoagulatorische Fähigkeit.

Faktor Va ist ein Cofaktor für die Faktor Xa-induzierte Aktivierung von Prothrombin zu Thrombin.

Faktor VIIIa ist der Cofaktor der Umwandlung von Faktor X zu Faktor Xa.

Wie bereits erwähnt, ist der Faktor Xa zusammen mit Faktor Va an der Umwandlung des Prothrombins zu Thrombin beteiligt. Demgemäß stellt die Aktivierung von Protein C in vivo zur Bildung von APC eine negative Feedback-Reaktion bei der Erzeugung von Thrombin auf dem Weg über den Abbau der Faktoren Va und VIIIa dar.

Zur Entwicklung der optimalen APC-Aktivität ist ein Cofaktor, Protein S, notwendig. Protein S ist ein nicht-enzymatischer Cofaktor für die Antikoagulations- und Pro-Fibrinolyse-Eigenschaften von APC. Im Plasma ist Protein S in verschiedenen Formen vorhanden. Zu diesen Formen gehört eine freie, aktive Form und eine inaktive Form, die mit dem C4b-binding-Protein nicht kovalent komplexiert ist. In einem Protein S-Mangelplasma kann APC seine normale Aktivität nicht ausüben. Erst nach Zugabe von Protein S erlangt das APC seine normale Aktivität wieder.

Dahlbäck et al. (siehe beispielsweise Dahlbäck et al., Proc. Natl.Acad.Sci. Vol. 90 (1993), p.1004-1008) haben bei Personen mit einer bisher unerklärlich hohen Neigung zu Thrombosen eine APC-Resistenz als eine Form der reduzierten APC-Response festgestellt.

Diese APC-Resistenz wurde ursprünglich aufgrund einer geringeren Verlängerung der aktivierten partiellen Thromboplastinzeit (aPTT) nach Zugabe von APC festgestellt.

Im speziellen ist heute bekannt, daß Mutationen im Faktor V, möglicherweise auch im Faktor VIII, speziell Mutationen in den Spaltstellen für APC, eine entscheidende Rolle für das Vorliegen einer APC-Resistenz spielen (Bertina R.M. et al., Nature, Vol. 369 (1994), p. 64-57).

Beispielsweise findet die proteolytische Inaktivierung von Faktor V durch APC an den Bindungsstellen $Arg^{306}$-$Gln^{307}$, $Arg^{505}$-$Gly^{506}$ und $Arg^{662}$-$Gln^{663}$ auf der schweren Kette statt, die leichte Kette des Faktor V wird bevorzugt

an den Bindungen Arg$^{1752}$-Arg$^{1753}$ oder Arg$^{1753}$-Ala$^{1754}$ gespalten. Eine Mutation des Faktor V an einer APC-Spaltstelle resultiert in einer erhöhten pro-koagulatorischen Aktivität des Faktor V. Damit stellt das Vorliegen einer Mutation im Faktor V für ein solches Individuum ein potentielles Risiko für das Auftreten thrombotischer Ereignisse dar.

Eine relativ häufig vorkommende Punkt-Mutation des Faktor V befindet sich im Protein an der Position 506, an der Arginin gegen Glutaminsäure ausgetauscht ist (siehe z.B. Bertina R.M. et al., Nature, Vol. 369 (1994), p. 64-57 und Kalafatis M. et al., J. Biol. Chem. Vol. 270, No. 8 (1995), p. 4053-57). Diese Faktor V-Mutation ist als Faktor V-Leiden bekannt und stellt eine der hauptsächlichen Ursachen für eine verminderte APC-Response, also eine geringere Verlängerung der Blutgerinnungszeit bei Zugabe von APC zu Testproben dar.

Eine APC-Resistenz kann vielfältige Auswirkungen haben, u.a. kann auch die funktionelle Protein S-Aktivität beeinflußt sein (siehe beispielsweise Faioni E.M. et al., Thromb.Haemost. 70 (6) (1993), p. 1067-1071; Faioni E.M. et al., Thromb.Haemost. 72 (4) (1994), p. 643-651 oder Moritz B. et al., Annals of Hematology Vol. 68, Suppl.II (1994), Nr. 267).

Aus der WO 93/10261 ist ein Test zur Diagnose thromboembolischer Erkrankungen bekannt, der auf der Bestimmung der APC-Response beruht. Hierfür werden nur solche Proben ausgewählt, bei denen geklärt ist, daß kein Protein S-Mangel vorliegt.

Von der Anmelderin ist in der EP-0 656 424 ein Test zur Bestimmung der APC-Response offenbart worden, welcher unter dem Namen IMMUNOCHROM® APC-Response Test erhältlich ist. Dieser Test beruht auf folgendem Testprinzip:

Verdünnte Plasmaproben, die als Quelle für Faktor VIII und APC Cofaktoren dienen, werden mit Faktor IXa, Faktor X, Phospholipiden, Ca$^{2+}$ und Spuren von Thrombin sowohl in Abwesenheit als auch in Anwesenheit von APC inkubiert. Unter Verwendung eines chromogenen Substrats wird die Menge an aktiviertem Faktor X bestimmt, und diese Menge an generiertem Faktor X ist der Faktor VIII-Konzentration proportional. Die Wirkung des APC bzw. der APC-Cofaktoren oder entsprechender Komplexe ist derart, daß der Faktor VIIIa teilweise inaktiviert wird.

Die APC-Response wird aus dem Verhältnis der Faktor Xa-Aktivität ohne und mit APC erhalten. Es gilt somit:

$$\text{APC-Response} = \frac{\Delta E405 \text{ (Mischung mit APC)}}{\Delta E405 \text{ (Mischung ohne APC)}}$$

Aus der WO 95/29259 ist bekannt, Plasma hinsichtlich dem Vorliegen von Faktor V-Mutationen mittels selektiver Hybridisierungstechniken auf Nukleinsäure-Ebene zu screenen.

Auch aus der WO 95/21938 ist bekannt, Mutationen betreffend Faktor V oder Faktor VIII mittels genetischer Methoden zu bestimmen.

Ein Defekt, speziell bezogen auf Faktor V, kann entweder genotypisch, also als Veränderung im Genom vorhanden, aber äußerlich ohne Auswirkungen, oder phänotypisch vorliegen. Weist ein Individuum beispielsweise einen genotypischen Faktor V-Defekt auf, so wird dieser Defekt mittels der herkömmlich durchgeführten Tests, beispielsweise mittels Globalbestimmungstests, nicht erkannt, und die Person gilt als klinisch gesund.

Die Individuen können bezüglich der Faktor V-Mutation heterozygot oder homozygot sein. Bei Vorliegen eines homozygoten Defekts ist dann die APC-Response wesentlich stärker vermindert, als dies bei einem heterozygot vorliegenden Defekt der Fall ist.

Im Gegensatz zu den Inhibitor-Defekten betreffend beispielsweise Antithrombin III, Protein C oder Protein S, die mit einer Häufigkeit von unter 1% auftreten, wurde gerade in den vergangenen Jahren auch bei einer gesunden, klinisch normalen Spenderpopulation eine Resistenz gegenüber aktiviertem Protein C gefunden, die als sogenannte APC-Resistenz, APC-Resistance oder APC-Response in die Literatur eingegangen ist.

Die Inzidenz einer APC-Resistenz bei Thrombose-Patienten liegt zwischen 10 bis 20%. Die Inzidenz einer Faktor V-Leiden Mutation in europäischen, klinisch gesunden Personen liegt zwischen etwa 2 bis 5% und ist massenabhängig.

Wie bereits erwähnt, sind zum Zwecke der Richtigkeits- und Präzisionsfindung bereits gut standardisierte Plasmen kommerziell erhältlich. Diese Standardplasmen werden durch sorgfältiges Screening der Einzelplasmen mittels Globaltests, wie z.B. aktivierte partielle Thromboplastinzeit-Messungen (aPTT) oder Bestimmung der Thromboplastinzeit (TPZ), erhalten. Neben diesen Screening-Tests werden auch verschiedene Sicherheitstests zur Testung auf gegebenenfalls vorhandene Pathogene, beispielsweise Tests auf das Vorhandensein von Viren, durchgeführt.

Speziell für die Herstellung sogenannter Normalplasmen ist es von großer Bedeutung, gut standardisierte Plasmapools zur Verfügung zu haben, die hinsichtlich aussagekräftiger Kriterien selektiert worden sind. Zu diesem Zwecke werden ausschließlich Globaltests wie aPTT oder TPZ durchgeführt. Defekte in Einzelplasmen, welche durch die Globaltests nicht bestimmt werden können, haben im allgemeinen keinen bzw. einen geringen Einfluß auf den Gesamtpool. Daher wurde bislang auch vermutet, daß eine reduzierte APC-Response in einer einzelnen Plasmaprobe keine Auswirkungen für die Herstellung von Normalplasmen zeigt.

Es wurde nun überraschenderweise gefunden, daß gerade für die Herstellung eines Normalplasmapools bereits ein Anteil von 5% Einzelplasmen mit reduzierter APC-Response zu einer signifikanten Fehleinschätzung von bestimmten Parametern bei Untersuchungsverfahren unter Verwendung des Normalplasmapools führen können.

Die vorliegende Erfindung stellt sich deshalb die Aufgabe, neue Selektionskriterien bezüglich der Herstellung von standardisierten Plasmapools zur Verfügung zu stellen und damit die Relevanz der Diagnoseverfahren zu erhöhen.

Diese Aufgabe wird erfindungsgemäß durch den Gegenstand der vorliegenden Patentansprüche gelöst.

Gemäß der vorliegenden Erfindung wird ein Verfahren zur Herstellung eines standardisierten Plasmapools für diagnostische Zwecke zur Verfügung gestellt, welches ein bisher nicht erkanntes und äußerst entscheidendes Selektionskriterien für Plasmen beinhaltet.

Das erfindungsgemäße Verfahren ist dadurch gekennzeichnet, daß Plasmaspenden bzw. Plasmaspender auf ihre APC-Response untersucht werden und der Plasmapool mit den Plasmaspenden bzw. Plasmen von Spendern, die eine normale APC-Response aufweisen, zur Herstellung des Plasmapools ausgewählt werden.

Unter APC-Response ist die Wirkung von APC auf das Blutgerinnungssystem zu verstehen. Entspricht der Effekt des zugesetzten APC in der zu untersuchenden Probe dem Effekt, der durch dieselbe Menge an APC in Normalplasma, welches als Standard eingesetzt wird, hervorgerufen wird, so liegt eine normale APC-Response vor. Das Symptom einer verringerten APC-Response ist durch eine geringere anti-koagulatorische Wirkung von APC auf die Probe charakterisiert.

Für Gerinnungsparameter wird der Normalbereich durch Bestimmung von wenigstens 50 Proben, besser 100 Proben, aus einem Normalkollektiv klinisch gesunder Personen ermittelt.

Für die statistische Auswertung ist die Art der Verteilung des Normalbereichs von Bedeutung. Der Normalbereich kann entweder gemäß einer Gauß schen Kurve normalverteilt sein und wird dann als Mittelwert mit einem +/-2 SD angegeben, oder er ist nichtnormalverteilt und der Wert wird als 95% Percentil angegeben.

Unter "normaler APC-Response" wird erfindungsgemäß ein Wert verstanden, der innerhalb dieses Normalbereichs, gegebenenfalls mit plus 5% Borderline, liegt.

Die APC-Response kann durch Anwendung verschiedener Tests bestimmt werden, vorzugsweise wird die APC-Response mit einem funktionellen Test bestimmt. Der Test ist beispielsweise ausgewählt aus der Gruppe von Faktor VIII-Inhibierungstest, aktivierte partielle Thromboplastinzeit-bestimmung (aPTT) und Thromboplastinzeitbestimmung (TPZ).

Alle Tests sind aus dem Stand der Technik seit langem bekannt und werden mittels der hierfür gängigen Methoden durchgeführt, mit dem Unterschied daß bei Durchführung des jeweiligen Tests APC zugesetzt wird.

Der Test zur Bestimmung der APC-Response ist vorzugsweise der Faktor VIII-Inhibierungstest gemäß der EP-0 656 424 (IMMUNOCHROM® APC-Response-Test von IMMUNO AG, Österreich), der auf einer kinetischen Methode basiert und bereits im Stand der Technik beschrieben worden ist.

Für das Auswahlkriterium der Plasmaspende bzw. Plasmaspender liegt die normale APC-Response bei Anwendung des IMMUNOCHROM® APC-Response-Tests nach der kinetischen Methode vorzugsweise innerhalb des Bereichs von 2,0 bis 3,7. Dieser Bereich entspricht etwa einem Wert von 70 bis 140% des Mittelwertes von mindestens 30, vorzugsweise mindestens 100 Einzelplasmaspenden.

Bei Verwendung anderer Tests ergeben sich die Bereiche für eine normale APC-Response nach entsprechenden, dem Fachmann bekannten Umrechnungen.

Die Bestimmung der APC-Response ist gegebenenfalls auch mittels eines immunologischen Testsystems möglich, z.B. unter Verwendung von anti-Faktor V-Leiden-Antikörpern, die die Mutation erkennen, bzw. Antikörpern gegen einen Bereich des Faktor V, der eine Spaltstelle für APC enthält.

Die APC-Response ist weiters bevorzugt durch einen Test zur genetischen Bestimmung einer Mutation eines Gerinnungsfaktors, beispielsweise von Faktor V-Leiden, bestimmbar, und die normale APC-Response ist durch das Fehlen der Mutation definiert.

In einer bevorzugten Ausführungsform wird die APC-Response in Einzelspenden untersucht. Sie kann auch in Minipools von 2 bis 100 Einzelspenden, vorzugsweise einem Minipool bis zu 10 Einzelspenden, am meisten bevorzugt bis zu 5 Einzelspenden, untersucht werden.

Der Plasmapool wird vorzugsweise lyophilisiert. Die Verfahrensschritte zur Fertigstellung des Plasmapools umfassen daher bevorzugt eine Lyophilisierung. Am meisten bevorzugt wird der Plasmapool in Abfülleinheiten zu jeweils weniger als 5 ml, vorzugsweise 0,5 bis 3 ml, abgefüllt und lyophilisiert. Allgemein gilt, daß beim Lyophilisationsschritt die Aktivität dieser Pools nur diesen Produktionsschritten entsprechend von der ursprünglichen Aktivität abweichen kann.

Mittels des erfindungsgemäßen Verfahrens ist es möglich, einen standardisierten Plasmapool herzustellen. Unter Normalplasma wird definitionsgemäß ein Plasma verstanden, welches definierte Eigenschaften bzw. definierte Gehalte an bestimmten Substanzen, insbesondere eine gleichmäßige Verteilung der Plasmafaktoren und der Inhibitoren, aufweist mit einer Aktivität von 100% der Norm.

Weiters kann es sich bei dem standardisierten Plasmapool beispielsweise um einen Referenz- oder um einen Kontrollplasmapool handeln. Aus diesen standardisierten Pools können dann auch standardisierte Mangelplasmapools bzw. Mangelplasmen hergestellt werden.

Einem Mangelplasma fehlt eine ganz bestimmte Substanz, beispielsweise ist ein Faktor VIII-Mangelplasma frei von Faktor VIII. Die entsprechende Substanz wird mittels hierfür gängige Methoden selektiv entfernt, beispielsweise durch Immundepletion. Durch Verwendung solcher Mangelplasmen ist es u.a. möglich, gezielt das Fehlen eines Gerinnungsfaktors oder eines entsprechenden Inhibitors in einer Patientenprobe zu bestimmen und das Mangelplasma wird als Reagens zur Bestimmung des entsprechenden, im Reagens fehlenden Stoffes in einer Probe eingesetzt.

Ein Kontroll- oder Referenzplasma dient der Richtigkeitskontrolle und stellt ein kalibriertes Plasma dar. Es enthält eine genau definierte Menge an einer oder mehreren Substanzen. So können für die Herstellung eines Kontrollplasmas beispielsweise ganz definiert die Faktoren des Prothrombinkomplexes abgetrennt worden sein, während alle anderen Faktoren gemäß einer normalen Verteilung in Plasma vorhanden sind.

Ein Kontrollplasma kann auch aus verschiedenen, vordefinierten Mischungen von Gerinnungsfaktoren, Fibrinolysefaktoren bzw. Inhibitoren bestehen, jeder Wert größer 0%, vorzugsweise 0 bis 300%, am meisten bevorzugt 1 bis 150% an einem der Faktoren kann eingestellt werden.

In dem erfindungsgemäßen Verfahren können Schritte zum Einstellen des angestrebten Wertes für einen Gerinnungsfaktor, Fibrinolysefaktor, und/oder deren Inhibitor vorgesehen werden. Der Gerinnungsfaktor, Fibrinolysefaktor, und/oder deren Inhibitor in den Plasmaspenden bzw. Plasmaspendern wird untersucht, und bestimmte Werte werden beispielsweise zur Herstellung eines entsprechenden Kontrollplasmas selektiert. Solche Kontrollplasmen sind dann beispielsweise auch zur Herstellung von Bezugskurven verwendbar.

Der Gerinnungsfaktor, Fibrinolysefaktor, oder deren Inhibitor wird in einer bevorzugten Ausführungsform aus dem Plasmapool entfernt, und man erhält dann ein entsprechendes Mangelplasma. Der entsprechende Gerinnungsfaktor, Fibrinolysefaktor, und/oder deren Inhibitor kann aber dem Plasmapool auch zugesetzt werden, und man erhält dann beispielsweise ein entsprechendes Referenz- oder Kontrollplasma.

Der Gerinnungsfaktor kann beispielsweise Faktor III, Faktor IV, Faktor VI, VII, V, II oder I sein. Als Fibrinolysefaktoren kommen z.B. Protein C, Protein S, Plasminogen oder tPA in Frage. Inhibitoren der Gerinnung und der Fibrinolyse können bespielsweise Antithrombin III PAi-1, Protein C Inhibitor (PCi) oder C1-Esterase Inhibitor sein.

In einer bevorzugten Ausführungsform wird ein Plasmapool hergestellt, an dem weiters Verfahrensschritte zur Erfassung von einzelnen bzw. der Summe von Gerinnungsaktivitäten, z.B. durch die Untersuchung der Thromboplastinzeit bzw. der aktivierten partiellen Thromboplastinzeit vorgenommen werden. Es werden dann diejenigen Plasmaspenden ausgewählt, welche Werte im Normalbereich ergeben, und der Plasmapool wird dann als Normalplasma fertiggestellt.

Die entsprechenden Werte für die Thromboplastinzeit liegen dabei vorzugsweise im Bereich von 60 bis 150% des Normalwertes. Die Werte für die aktivierte partielle Thromboplastinzeit liegen bevorzugterweise in einem Bereich zwischen 27,6 bis 34,3 s (gemessen mittels DAPTTIN® auf Electra 1600).

Ergänzend können auch Verfahrensschritte zur Untersuchung der viralen Kontaminationen der Plasmaspenden bzw. Plasmaspender vorgenommen werden. Es werden dann bevorzugterweise diejenigen Plasmaspenden bzw. Plasmaspender für die Herstellung des Plasmapools selektiert, welche virussicher sind bzw. werden jene Spender ausgewählt, die nicht mit Viren infiziert sind. Beispielsweise werden Antigen- bzw. Antikörper-Bestimmungen auf Hepatitis B, Hepatitis C und HIV durchgeführt.

Die Untersuchung solcher viraler Kontaminationen kann auch mittels weiterer hierfür gängiger Methoden erfolgen, beispielsweise mittels einer Nukleinsäure-Amplifikation wie der PCR-Methode.

Als virussicher sind dann diejenigen Plasmen anzusehen, bei denen keine Viren nachweisbar sind, d.h. daß der Gehalt an Viren unter der Nachweisgrenze liegt.

Mittels des erfindungsgemäßen Verfahrens sind damit verschiedene standardisierte Plasmapools als neue Qualitätsprodukte erhältlich. Dieses Qualitätsprodukt aus mindestens 1000 Einzelplasmaspenden, das eine Serie von mindestens 10 Plasmapools bestehend aus mindestens 10 Einzelplasmaspenden umfaßt, ist dadurch gekennzeichnet, daß jeder Pool eine normalen APC-Response aufweist. Diese normale APC-Response im Qualitätsprodukt kann beispielsweise durch einen Wert im Bereich von 2,5 bis 3,0 im IMMUNOCHROM® APC Response-Test (kinetische Methode) gemäß der EP-0 656 424 ausgedrückt werden.

Vorzugsweise sind die erfindungsgemäßen Qualitätsprodukte aus mindestens 10 000 Einzelplasmaspenden zusammengesetzt. Gemäß der vorliegenden Erfindung werden also Produkte zur Verfügung gestellt, die über Jahre und Jahrzehnte die Sicherheit der gleichbleibenden Qualität aufweisen. Die erfindungsgemäßen Qualitätsprodukte zeichnen sich daher durch eine extrem hohe Qualitätssicherheit aufgrund des neuen Selektionskriteriums aus.

Gemäß einem weiteren Aspekt der vorliegenden Erfindung wird auch ein humanes Referenzplasma für diagnostische Zwecke zur Verfügung gestellt, welches einen normalen Faktor V-Gehalt aufweist und Liganden für den Faktor V, welche an Arginin$^{506}$ des Faktor V binden.

Der Ligand besitzt die Fähigkeit, den Faktor V in Kalibratoren und Kontrollen spezifisch vor einer Inaktivierung durch aktives Protein C zu schützen. Damit wird im Kalibrator bzw. in der Kontrolle für den Faktor V ein Zustand erreicht, der eine reduzierte APC-Response verursacht. Dieses entspricht dem Zustand, wie er im Plasma von Patienten auftritt, die im Gen des Faktor V die für den Faktor V-Leiden typische Mutation (Arg$^{506}$Gln) besitzen. Da diese nur selten homozygot auftritt, ist es sehr aufwendig, Plasma für die Herstellung entsprechender Kalibratoren und Kontrollen von solchen Patienten zu gewinnen. Gemäß der vorliegenden Erfindung ist es nun möglich, entsprechende Faktor V enthaltende Referenzplasmen und insbesondere ein "APC response 0%", "APC response 50%" bzw. "APC response 100%" Referenzplasma zur Verfügung zu stellen.

Der Ligand ist eine Struktur mit einer hohen Affinität bzw. Avidität zum Faktor V, speziell zu nicht mutiertem Faktor V, sodaß eine lang dauernde bzw. stabile Bindung zwischen Faktor V und dem entsprechenden Liganden ermöglicht wird. Bevorzugterweise wird als Ligand ein Peptid, ein Immunglobulin oder ein Proteinfragment eingesetzt. Durch bekannte Methoden der Peptidkombinatorik können derart spezifische Peptide oder Proteinfragmente routinemäßig und sehr spezifisch für den Faktor V hergestellt werden. Die Auswahl eines geeigneten Liganden erfolgt in der Praxis beispielsweise derart, daß aus einem physiologischerweise gebundenen Liganden oder Reaktionspartner der Bindungsbereich zu Faktor V-Protein ausgewählt wird. Diese geeignete Bindungsstelle kann beispielsweise mittels eines Peptid-Scans, wie in den Beispielen noch näher erläutert, ermittelt werden. Gleichwohl können aber auch Liganden zum Einsatz kommen, die organisch chemische Substanzen darstellen, welche die physiologische Raumstruktur eines entsprechenden Liganden nachbilden können. Solche Substanzen können beispielsweise Zuckerverbindungen, Nukleotide oder organische Ringverbindungen sein.

Eine bevorzugte Möglichkeit zur "Konstruktion" eines solchen Liganden besteht darin, bestimmte Peptide oder modifizierte Peptide von einem physiologischen Bindungsprotein zum Faktor V-Protein abzuleiten. Der Ligand kann beispielsweise von einem Protein, welches Faktor V als Substrat erkennt, abgeleitet sein, beispielsweise von einer Serin-Protease, wie vorzugsweise von aktiviertem Protein C (APC), Plasmin, Thrombin, Faktor Xa, Trypsin oder Chymotrypsin. Dieses physiologische Bindungsprotein für Faktor V kann ein solches sein, das zur Spaltung des Faktor V-Proteins bzw. des Faktor Va-Proteins führt, es kann aber auch ein solcher Bindungspartner sein, der nicht zur Spaltung des entsprechenden Faktor V-Moleküls führt.

Wenn der Ligand ein Peptid ist, so umfaßt es wenigstens drei Aminosäuren, wobei gemäß einer bevorzugten Ausführungsform wenigstens eine Aminosäure unphysiologisch ist. Beispielsweise umfaßt der Ligand maximal 200 Aminosäuren, bevorzugt 3 bis 100 Aminosäuren, am meisten bevorzugt 5 bis 20 Aminosäuren.

Das Referenzplasma enthält vorzugsweise eine bestimmte Menge an Peptiden, welche mindestens 10%, vorzugsweise 15%, am meisten bevorzugt 25% homolog zum aktiven Zentrum von aktiviertem Protein C sind.

In einer weiteren bevorzugten Ausführungsform wird ein Peptid verwendet, welches ein Peptid ausgewählt aus der Gruppe bestehend aus Sequenznummer 1 bis 13 (SEQ ID NO: 1-13) umfaßt.

Vorzugsweise handelt es sich um ein Peptid, welches selektiv nicht mutierten Faktor V bindet.

Der Ligand kann ein poly- oder monoklonaler anti-Faktor-V-Antikörper sein oder auch ein Proteinfragment, beispielsweise ein entsprechendes Fab-Fragment, ein sogenannter "single-chain antibody" oder ein "mini-body".

Der Ligand im Referenzplasma kann in einer mehr als äquimolaren Menge enthalten sein, aber auch in einer äquimolaren oder weniger als äquimolaren Menge, beispielsweise in einer "halb-äquimolaren Menge". Die Menge an gebundenem Faktor V ist vorzugsweise standardisiert.

Der Ligand ist in einer weiteren bevorzugten Ausführungsform mit einem zur Detektion geeigneten Label versehen. Als Label kommen alle hierfür aus dem Stand der Technik geeigneten Label in Frage, beispielsweise ist der Label ausgewählt aus der Gruppe Chromogen, Fluorogen, Radionuklid, Enzym, Lanthanid und Luminogen.

Das Referenzplasma gemäß der vorliegenden Erfindung zeichnet sich insbesondere auch durch eine ausgezeichnete Lagerstabilität aus. Vorzugsweise wird es in lyophilisierter Form gelagert. Als ein Kriterium für die Lagerstabilität kann beispielsweise gelten, daß das entsprechende Referenzplasma, welches unter Verwendung eines gegebenenfalls mit einem Label versehenen Liganden hergestellt worden ist, nach 2 Jahren eine Änderung in der APC-Response von nicht mehr als 10 %, vorzugsweise nicht mehr als 5 %, aufweist.

Das erfindungsgemäße Referenzplasma wird vorzugsweise auf Basis eines erfindungsgemäß standardisierten Plasmapools hergestellt.

Weiters wird mit der vorliegenden Erfindung auch ein Verfahren zur Herstellung eines Referenzplasma zur Verfügung gestellt, wobei einem Normalplasma ein Ligand für den Faktor V zugesetzt wird, welcher nach den folgenden Kriterien ausgesucht ist:

a) der Ligand bindet an Arginin$^{506}$ des Faktor V,
b) der Ligand bindet an den Faktor V in kompetitiver Weise mit aktiviertem Protein C, und/oder
c) der Ligand hat eine höhere Affinität für den Faktor V als aktiviertes Protein C.

Die vorliegende Erfindung soll im folgenden noch durch Beispiele näher erläutert werden, ohne diese jedoch darauf zu beschränken.

## Beispiel 1: Herstellung und Testung der Plasmapools

Ein Normalplasmapool (NPP) wurde aus 25 Plasmaspenden mit abgeklärter normaler APC-Response, auf Grundlage einer gesicherten Abwesenheit einer APC-Resistenz aufgrund des Fehlens einer Faktor V-Leiden-Mutation, erhalten. Aus abnormen Plasmen mit abgeklärter APC-Resistenz, also dem gesicherten Vorliegen einer Faktor V-Leiden-Mutation, wurden entsprechende standardisierte Referenzplasmapools hergestellt. Das Fehlen oder Vorliegen einer Faktor V-Leiden-Mutation wird mittels einer modifizierten PCR-Methode (nach Bertina et al., Nature, Vol. 369 (1994), p. 64-57) festgestellt. Die Normalplasmapools wurden dann mit unterschiedlichen Mengen der standardisierten Referenzplasmapools gemischt, und die verschiedenen Mischungen wurden dann mittels unterschiedlicher Tests untersucht.

In Tabelle 1 sind die Ergebnisse angeführt. Folgende Tests wurden durchgeführt:

Spalte 2:    APC-Response-Ratio über chromogene Faktor VIII-Bestimmung mittels IMMUNOCHROM® APC-Response

Spalte 3:    APC-aPTT gemäß einer modifizierten Methode von Dahlbäck, PNAS 90 (1993), p. 1004-1008

Spalte 4:    Protein S IL-Bestimmung über Protein S sensitives bovines Thromboplastin nach Preda et al., Thrombos. Res. 60 (1990), p. 19-32

Spalte 5:    Protein S-Bestimmung mit einem modifizierten Test nach Comp et al., J.Clin.Invest. 74 (1984), p. 2082-2088

**Ergebnisse:**

Alle Testergebnisse zeigen deutlich, daß bei Vorliegen einer Mischung von 95% NPP, also einem 5%-igen Anteil abnormaler Plasmen am Gesamtplasmapool, besonders bei den als APC-Response-Ratio und APC-aPTT dargestellten Werte, bereits deutlich von dem Wert des Normalplasmapools abweichen.

Deutlich zu sehen ist auch der Einfluß auf die Bestimmung der Protein S- Aktivität.

Tabelle 1:

| Probe (in % NPP) | APC-Response Ratio | APC aPTT Ratio | Protein S "IL" % Aktivität | Protein S modifiziert nach Comp |
|---|---|---|---|---|
| 100 % NPP | 2,60 | 3,67 | 103 | 105 |
| 95% | 2,47 | 3,37 | 106 | 110 |
| 90% | 2,53 | 3,15 | 101 | 107 |
| 80% | 2,28 | 2,84 | 98 | 99 |
| 60% | 2,14 | 2,58 | 91 | 100 |
| 40% | 1,97 | 2,32 | 78 | - |
| 20% | 1,88 | 2,04 | 69 | 78 |
| 10% | 1,82 | 1,89 | 66 | 78 |
| 5% | 1,76 | 1,94 | 64 | 80 |
| 0% | 1,73 | 1,91 | 62 | 80 |

**Beispiel 2:**

**Auswirkung APC-resistenter Plamapools auf die Protein S-Bestimmung**

Wie aus Beispiel 1 und Tabelle 1 erkenntlich, kann das Vorhandensein von APC-resistenten Einzelplasmen in einem Plasmapool für ein Protein S-Mangelplasma enorme Konsequenzen haben.

Protein S-Mangelplasma wird üblicherweise als Pool in der Größe von etwa 10-20 Einzelplasmaspenden hergestellt. Wird Protein S-Mangelplasma beispielsweise aus einem Plasmapool hergestellt, der lediglich zu 40% aus Normalplasma und zu 60% aus APC-resistenten Einzelplasmen besteht, so führt die Bestimmung von Protein S in einer Patientenprobe unter Verwendung dieses Mangelgelplasmas als Reagens zu einer groben Fehleinschätzung des tatsächlichen Protein S-Gehalts. Statt in der Patientenprobe einen Normalgehalt an Protein S zu finden, erhält man fälschlicherweise einen wesentlich geringeren Wert. Der Patient würde dann fälschlicherweise als Protein S-Mangelpatient eingestuft werden und hätte sich unnötigerweise einer entsprechenden Therapie zu unterziehen.

Es ist also überraschenderweise mittels des erfindunggemäßen Verfahrens sehr gut möglich, diagnostische Fehler aufgrund einer Verwendung von ungenügend standardisierten Reagentien zu vermeiden.

### Beispiel 3:

#### a) Auswahl von Peptiden aus dem Protein C, die den Faktor V binden

Zur Identifikation von Peptiden, die den Faktor V binden, wurde ein Peptid-Scan über die gesamte Aminosäuresequenz des Protein C mittels gängiger Methoden der Peptidchemie durchgeführt. Hierfür wurden Peptide à 13 Aminosäuren (AA) um jeweils 4 AA versetzt und auf einem Papierträger kovalent gebunden angefertigt. Dieser Träger wurde nun mit biotinyliertem Faktor V inkubiert. Das Biotin wurde entsprechend gängiger Methoden mit einem mit alkalischer Phosphatase markierten Streptavidin und 5-Bromo-4-Chloro-3-Indolyl-Phosphat (BCIP) als Farbstoff nachgewiesen.

Der Nachweis der Bindung erfolgte auch durch Inkubation der Peptidscanmembran mit Vollplasma bzw. Faktor V-Mangelplasma (immunadsorbiert) sowie indirekt mit einem Kaninchen-anti-human-Faktor-V-Antikörper, der mit einem mit alkalischer Phosphatase markierten Schwein-anti-Kaninchen-Antikörper reagieren konnte.

Folgende Sequenzen wurden als Faktor-V-stark-bindend gefunden (AA von Signalsequenz 1-32 gezählt):

$$\text{AA } 265\text{--}277 \qquad (\text{LEGYDLRRWEKWE}) \quad (\text{SEQ ID NO: 2})$$

$$\text{AA } 409\text{--}422 \qquad (\text{HNYGVYTKVSRYL}) \quad (\text{SEQ ID NO: 3})$$

Folgende Sequenzen wurden als Faktor-V-schwach-bindend identifiziert (AA von Signalsequenz 1-32 gezählt):

$$\text{AA } 217\text{--}229 \qquad (\text{MTRRGDSPWQVVL}) \quad (\text{SEQ ID NO: } 12)$$
$$\text{AA } 339\text{--}358 \qquad (\text{TGWGYHSSREKEAKRNRTF}) \ (\text{SEQ ID NO: } 13)$$

Negative Kontrollen wurden nicht gebunden.

Im weiteren wurden ein entsprechend biotinyliertes Faktor V-Peptid (KSRSDRRGIQRAAD-Bio; SEQ ID NO: 14), ein biotinyliertes Faktor V-Leiden-Peptid (KSRSDR**Q**GIQRAAD-Bio; SEQ ID NO: 15) sowie ein Kontrollpeptid (KSRSDRRGI**G**RAAD-Bio; SEQ ID NO: 11) mit einer Mutation außerhalb der Position R506 und einer starken Bindung zum Protein C für den Nachweis weiterer bindender Sequenzen eingesetzt. Der Bindungsnachweis erfolgte, wie bereits beschrieben, mit Streptavidin-alkalischer Phosphatase.

Dabei konnten folgende Sequenzen als "Faktor-V-Peptid-starkbindend" gefunden werden (AA von Signalsequenz 1-32 gezählt):

$$\text{AA } 4\text{--}16 \qquad (\text{LLTSLLLFVATWGI, Signalsequenz; SEQ ID NO: } 6)$$
$$\text{AA } 73\text{--}85 \qquad (\text{FQNVDDTLAFWSK; SEQ ID NO: } 4)$$
$$\text{AA } 166\text{--}178 \qquad (\text{YKLGDDLLQCHP; SEQ ID NO: } 7)$$
$$\text{AA } 279\text{--}304 \qquad (\text{LDIKEVFV}\textbf{HPNYSKS}\text{TTDNDIALLH}) \text{ mit der Konsensus-}$$
$$\text{sequenz: AA } 288\text{--}293 \ \textbf{HPNYSKS} \quad (\text{SEQ ID NO: } 8)$$
$$\text{AA } 333\text{--}346 \qquad (\text{GQETLVTGWGYHS; SEQ ID NO: } 5)$$
$$\text{AA } 336\text{--}352 \qquad (\text{TLVTGWGYHSSREKEA; SEQ ID NO: } 9))$$
$$\text{AA } 427\text{--}439 \qquad (\text{GLLHNYGVYTKVS; SEQ ID NO: } 1)$$
$$\text{AA } 449\text{--}461 \qquad (\text{IRDKEAPQKSWAP; SEQ ID NO: } 10, \text{ C-Terminus})$$

Das Faktor V-Kontrollpeptid KSRSDRRGI**G**RAAD-Bio (SEQ ID NO: 11) wurde ebenfalls mit den Protein C-Sequenzen zur Reaktion gebracht.

Dabei konnten folgende Sequenzen zusätzlich als Faktor-V-stark-bindend gefunden werden (AA von Signalse-

quenz 1-32 gezählt):

AA 265-277 (LEGYDLRRWEKWE; SEQ ID NO: 2)

Folgende Sequenzen wurden als Faktor-V-schwach-bindend identifiziert (AA von Signalsequenz 1-32 gezählt):

AA 336-352 (TLVTGWGYHSSREKEA; SEQ ID NO: 9)

Vier Protein C-Peptide konnten identifiziert werden, die entweder nur mit dem Faktor V-, nicht aber mit dem Faktor V-Leiden-Peptid reagieren (beispielsweise ein Peptid mit der Sequenz GLLHNYGVYTKVS; SEQ ID NO: 1), oder die nur mit dem Faktor V-Leiden- und nicht mit dem Faktor V-Peptid reagieren (beispielseise Peptide mit den Sequenzen GDDLLQCHPAVKF; SEQ ID NO: 16, DGKMTRRGDSPWQ; SEQ ID NO: 17 oder GDSGGPMVASHG; SEQ ID NO: 18).

**b) Herstellung von in plasmahaltigen Lösungen stabilen Peptiden**

Da Peptide durch die stets im Plasma vorhandenen proteolytischen Aktivitäten relativ schnell abgebaut werden, wird erfindungsgemäß ein stabiles Peptid dadurch erzeugt, daß statt den physiologischen L-Aminosäure-Precursoren mit bekannten Methoden der Peptidchemie D-Aminosäure-Precursoren für die Peptidsynthese eingesetzt werden.

**c) Herstellung eines Kalibrators "APC response 0%"**

Faktor V hat das Molekulargewicht 333 000, die Konzentration in Plasma beträgt 10 µg/ml. Dies entspricht einer molaren Konzentration von 0,03 µmol/l. Das Peptid enthaltend die Sequenz GLLHNYGVYTKVS; SEQ ID NO: 1, welches nur Faktor V-, nicht aber Faktor V-Leiden bindet (siehe Beispiel 3a), wird dem Normalplasapool in einer Konzentration von 0,06 µmol/l, also in molarem Überschuß zugesetzt.

Dieses Kalibrator-Bulkmaterial wird mit einem Stabilisator (10 Vol% HEPES/Glycin-Lösung) versetzt und zu 1,1 ml abgefüllt, lyophilisiert und als Kalibrator "APC response 0%" zur Verfügung gestellt. Der Kalibrator kann in 1 ml destilliertem Wasser rekonstituiert und dann verwendet werden.

**d) Kalibratorset**

Das in Beispiel 3c) eingesetzte Peptid wird in einer Konzentration von 0,35 µmol/l dem Normalplasmapool zugegeben, so daß ein "Kalibrator-Bulkmaterial 0%" erhalten wird. Der Normalplasmapool dient als Kalibrator-Bulk 4. Durch geeignete Mischung der Kalibrator-Bulks 1 und 4 (Mischungsverhältnis 1+2 bzw. 2+1) werden die Kalibratoren 2 und 3 hergestellt.

Die Kalibrator-Bulks werden mit einem Stabilisator (10 Vol% HEPES/Glycin-Lösung) versetzt und zu 1,1 ml abgefüllt. Die Rekonstitution der Plasmen des Kalibratorsets erfolgt in 1 ml destilliertem Wasser.

## Patentansprüche

1. Verfahren zur Herstellung eines standardisierten Plasmapools für diagnostische Zwecke, dadurch gekennzeichnet, daß Plasmaspenden bzw. Plasmaspender auf ihre APC-Response untersucht werden und der Plasmapool mit den Plasmaspenden bzw. Plasmen von Spendern, die eine normale APC-Response aufweisen, hergestellt wird.

2. Verfahren nach Anspruch 1, dadurch gekennzeichnet, daß die APC-Response mit einem funktionellen Test bestimmt wird, wobei die normale APC-Response durch einen Wert innerhalb des Normalbereiches definiert wird.

3. Verfahren nach Anspruch 1 oder 2, dadurch gekennzeichnet, daß der Test zur Bestimmung des APC-Response ausgewählt ist aus der Gruppe bestehend aus Faktor VIII-Inhibierungstest, aktivierter partieller Thromboplastinzeitzeitbestimmung und Thromboplastinzeitbestimmung.

4. Verfahren nach einem der Ansprüche 1 bis 3, dadurch gekennzeichnet, daß als Test zur Bestimmung der APC-Response der APC-Response-Test gemäß der EP-0 656 424, vorzugsweise nach der kinetischen Methode, durchgeführt wird und die normale APC-Response durch einen Wert innerhalb des Bereiches von 2,0 bis 3,7 definiert wird.

5. Verfahren nach einem der Ansprüche 1 bis 4, dadurch gekennzeichnet, daß die APC-Response mit einem Test zur Bestimmung einer Mutation eines Gerinnungsfaktors, bespielsweise Faktor V-Leiden, bestimmt wird, und die normale APC-Response durch das Fehlen der Mutation definiert wird.

6. Verfahren nach einem der Ansprüche 1 bis 5, dadurch gekennzeichnet, daß die APC-Response in Einzelspenden untersucht wird.

**7.** Verfahren nach einem der Ansprüche 1 bis 6, dadurch gekennzeichnet, daß die APC-Response in Minipools von 2 bis 100 Einzelspenden, vorzugsweise bis 10 Einzelspenden, am meisten bevorzugt bis 5 Einzelspenden untersucht wird.

**8.** Verfahren nach einem der Ansprüche 1 bis 7, dadurch gekennzeichnet, daß es einen Lyophilisierungsschritt umfaßt.

**9.** Verfahren nach Anspruch 8, dadurch gekennzeichnet, daß der Plasmapool in Behältern zu jeweils weniger als 5 ml, vorzugsweise 0,5 bis 3 ml, abgefüllt und lyophilisiert wird.

**10.** Verfahren nach einem der Ansprüche 1 bis 9, dadurch gekennzeichnet, daß Verfahrensschritte zum Einstellen eines bestimmten Wertes für mindestens einen Gerinnungsfaktor, Fibrinolysefaktor, oder deren Inhibitor vorgenommen werden.

**11.** Verfahren nach Anspruch 10, dadurch gekennzeichnet, daß der Plasmapool als Kontrollplasma fertiggestellt wird.

**12.** Verfahren nach Anspruch 10, dadurch gekennzeichnet, daß der Gerinnungsfaktor, Fibrinolysefaktor, oder deren Inhibitor aus dem Plasmapool entfernt wird.

**13.** Verfahren nach Anspruch 12, dadurch gekennzeichnet, daß der Plasmapool als Mangelplasma fertiggestellt wird.

**14.** Verfahren nach Anspruch 10 oder 11, dadurch gekennzeichnet, daß der Gerinnungsfaktor, Fibrinolysefaktor, und/oder deren Inhibitor dem Plasmapool zugesetzt wird.

**15.** Verfahren nach Anspruch 10, dadurch gekennzeichnet, daß der Gerinnungsfaktor, Fibrinolysefaktor, und/oder deren Inhibitor in den Plasmaspenden bzw. Plasmaspendern untersucht wird, und ein vordefinierter Wert zur Herstellung des entsprechenden Kontrollplasmas selektiert wird.

**16.** Verfahren nach einem der Ansprüche 1 bis 15, dadurch gekennzeichnet, daß weiters Verfahrensschritte zur Untersuchung der Thromboplastinzeit bzw. der aktivierten partiellen Thromboplastinzeit vorgenommen werden, und diejenigen Plasmaspenden ausgewählt werden, welche Werte im Normalbereich ergeben, und der Plasmapool als Normalplasma fertiggestellt wird.

**17.** Verfahren nach Anspruch 16, dadurch gekennzeichnet, daß diejenigen Plasmaspenden bzw. Plasmen von Spendern ausgewählt werden, die eine Thromboplastinzeit im Bereich von 60 bis 150% des Normalwertes aufweisen.

**18.** Verfahren nach Anspruch 16, dadurch gekennzeichnet, daß diejenigen Plasmaspenden bzw. Plasmen von Spendern ausgewählt werden, die eine aktivierte partielle Thromboplastinzeit im Bereich von 27,6 bis 34,4 s aufweisen.

**19.** Verfahren nach einem der Ansprüche 1 bis 18, dadurch gekennzeichnet, daß weiters Verfahrensschritte zur Untersuchung der viralen Kontamination der Plasmaspenden bzw. Plasmen von Spendern vorgenommen werden, und diejenigen Plasmaspenden bzw. Plasmaspender zur Herstellung-des Plasmapools selektiert werden, welche virussicher sind.

**20.** Qualitätsprodukt aus mindestens 1000 Einzelplasmaspenden, das eine Serie von mindestens 10 Plasmapools bestehend aus mindestens 10 Einzelplasmaspenden umfaßt, dadurch gekennzeichnet, daß jeder Pool eine normale APC-Response aufweist, ausgedrückt durch einen Wert im Bereich von 2,5 bis 3,0 im APC-Response-Test gemäß der EP-0 656 424, erhältlich durch ein Verfahren nach einem der Ansprüche 1 bis 19.

**21.** Qualitätsprodukt nach Anspruch 20, dadurch gekennzeichnet, daß es aus mindestens 10 000 Einzelplasmaspenden zusammengesetzt ist.

**22.** Humanes Referenzplasma für diagnostische Zwecke, dadurch gekennzeichnet, daß es einen normalen Faktor V-Gehalt aufweist und Liganden für den Faktor V, welche an Arginin[506] des Faktor V binden.

**23.** Referenzplasma nach Anspruch 22, dadurch gekennzeichnet, daß eine bestimmte Menge an Peptiden enthalten ist, welche mindestens 10%, vorzugsweise 15% und am meisten bevorzugt 25% homolog zum aktiven Zentrum von aktiviertem Protein C sind.

**24.** Referenzplasma nach Anspruch 23, dadurch gekennzeichnet, daß das Peptid ein Peptid ausgewählt aus der Gruppe bestehend aus Sequenznummer 1 bis 13 umfaßt.

**25.** Referenzplasma nach einem der Ansprüche 22 bis 23, dadurch gekennzeichnet, daß eine mehr als äquimolare Menge des Liganden enthalten ist.

**26.** Referenzplasma nach einem der Ansprüche 22 bis 25, dadurch gekennzeichnet, daß eine weniger als äquimolare Menge des Liganden enthalten ist und die Menge an gebundenem Faktor V standardisiert ist.

**27.** Referenzplasma nach einem der Ansprüche 22 bis 26, dadurch gekennzeichnet, daß der Ligand mit einem zur Detektion geeigneten Label versehen ist.

**28.** Referenzplasma nach einem der Ansprüche 22 bis 27, dadurch gekennzeichnet, daß es auf Basis eines standardisierten Plasmapools gemäß Anspruch 20 oder 21 hergestellt ist.

**29.** Verfahren zur Herstellung eines Referenzplasma nach einem der Ansprüche 22 bis 28, wobei einem Normalplasma ein Ligand für den Faktor V zugesetzt wird, welcher nach den folgenden Kriterien ausgesucht ist

a) der Ligand bindet an Arginin$^{506}$ des Faktor V,
b) der Ligand bindet an den Faktor V in kompetitiver Weise mit aktiviertem Protein C, und/oder
c) der Ligand hat eine höhere Affinität für den Faktor V als aktiviertes Protein C.

**30.** Verfahren nach Anspruch 29, dadurch gekennzeichnet, daß eine mehr als äquimolare Menge des Liganden zugesetzt wird.

**31.** Verfahren nach Anspruch 29, dadurch gekennzeichnet, daß eine geringere als äquimolare Menge des Liganden zugesetzt wird und der Anteil an gebundenem Faktor V definiert wird.

**32.** Verfahren nach einem der Ansprüche 29 bis 31, dadurch gekennzeichnet, daß das Normalplasma hergestellt ist nach einem der Ansprüche 1 bis 19.